(19) Europäisches Patentamt
European Patent Office
Office européen des brevets

(11) Publication number: 0 065 319 B1

(12) **EUROPEAN PATENT SPECIFICATION**

(45) Date of publication of patent specification: **17.10.84**

(51) Int. Cl.$^3$: **C 07 C 49/587,** C 07 C 45/72

(21) Application number: **82105240.4**

(22) Date of filing: **12.02.80**

(60) Publication number of the earlier application in accordance with Art. 76 EPC: **0 015 412**

(54) **Process for the preparation of an unsaturated macrocyclic ketone.**

(30) Priority: **12.02.79 US 11685**
**19.09.79 US 76960**

(43) Date of publication of application:
**24.11.82 Bulletin 82/47**

(45) Publication of the grant of the patent:
**17.10.84 Bulletin 84/42**

(84) Designated Contracting States:
**CH DE FR GB IT NL SE**

(56) References cited:
**FR-A-1 237 623**

**TETRAHEDRON LETTERS, no. 37, 1977, pages 3285-3286, Pergamon Press, G.B. J. TSUJI et al.: "Synthesis of cis-civetone from a butadiena telomer"**

**HELVETICA CHIMICA ACTA, vol. 62, no. 269, fasciculus 8, 1979, pages 2661-2672, Basel, CH. G. BUCHI et al.: "Macrocycles by olefination of dialdehydes with 1,3-bis(dimethyl-phosphono)-2-pro panone"**

(73) Proprietor: **FIRMENICH SA**
**Case postale 239**
**CH-1211 Genève 9 (CH)**

(72) Inventor: **Büchi, George H.**
**100 Memorial Drive**
**Cambridge, Mass. (US)**
Inventor: **Wüest, Hans**
**100 Memorial Drive**
**Cambridge, Mass. (US)**

(74) Representative: **Weinhold, Peter, Dr. et al**
**Siegfriedstrasse 8**
**D-8000 München 40 (DE)**

**EP 0 065 319 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Among the most appreciated musky ingredients known in the art of perfumery, certain macrocyclic ketones such as civetone as well as cyclopentadecanone and its higher methyl homologue, 3-methyl-cyclopentadecanone, better known under the name of EXALTONE® and muscone, respectively, have acquired a special renown.

A great number of synthetic methods have been proposed in the past for their preparation and a few of them have been applied for industrial production. In spite of this, owing to their high price these macrocyclic ketones have found a rather limited utilization and industry has spent a great deal of effort in devising more economical processes for their preparation [see e.g. the review in Cosmetics and Perfumery, *88*, 67 (1973)]. The present invention provides a new and original solution to this problem.

It is an object of the present invention to provide a process for the preparation of unsaturated macrocyclic ketones of formule

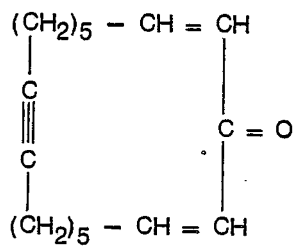

(I)

which process comprises reacting in the presence of a basic reagent a dialdehyde of formula

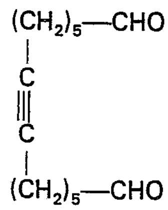

(II)

with a diphosphonate of formula

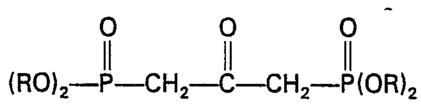

(III)

wherein each of symbols R represents a lower alkyl radical.

The symbol R in formula (III) represents a lower alkyl radical, e.g. methyl or ethyl. Compounds (III) can be obtained according to partially known synthetic methods, for instance in accordance with the method illustrated hereinbelow:

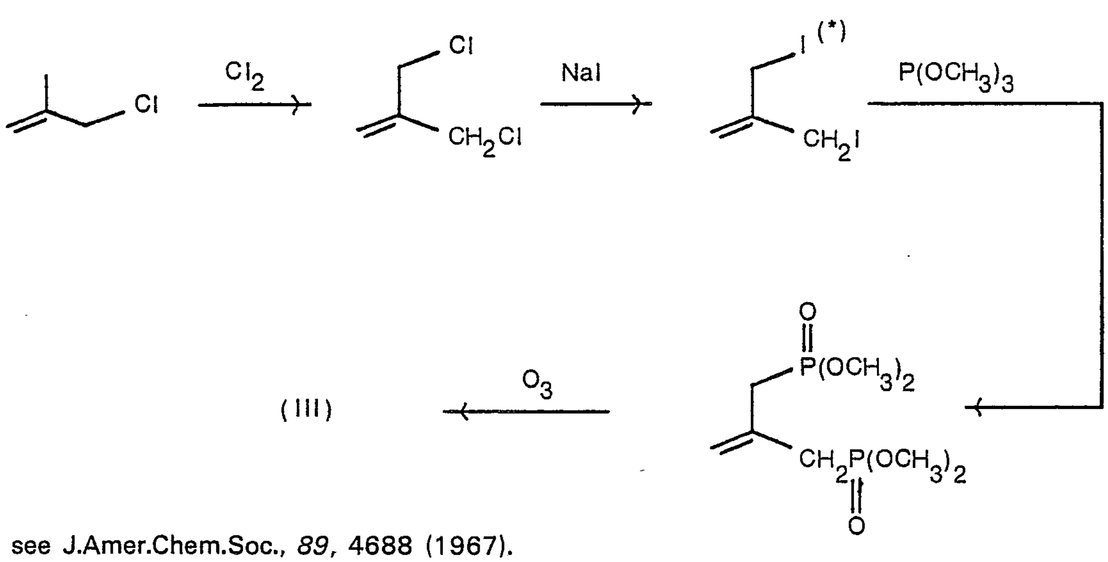


(*) see J.Amer.Chem.Soc., *89*, 4688 (1967).

The reaction between dialkyl-diphopsphonate (III) and dialdehyde (II) occurs, according to the invention, in the presence of a basic reagent. Useful basic reagents include organic or mineral bases. Good yields of final products are obtained by using an alkali metal hydride, carbonate or bicarbonate; sodium hydride or potassium bicarbonate are preferred. The reaction is preferably carried out in an inert organic solvent, or aqueous organic solvent, such as an alcohol, e.g. ter-butanol, or an ether such as tetrahydrofuran or diethylene glycol dimethyl ether.

The said reaction can be effected either in a single step or as a combined two-step synthesis. In this latter case the first step consists in the formation of a mono-phosphonate which is then converted into the desired ketone by means of a further treatment with a basic reagent.

Though the temperature does not play any major determinant role for the good course of the reaction, it has been found that for economical and practical reasons a temperature of between 50 and 100°C can be satisfactorily applied. In this range of temperatures the formation of by-products is substantially reduced and the reaction times are sufficiently short.

On the contrary, when the process of the invention is carried out according to the two step variant described above, the temperature applied to the first reaction step is lower that the limit indicated.

Preferably, it is 20—30°C, whereas the subsequent conversion is effected at a temperature of between 50 and 100°C.

The above described dienynone (I) is a useful starting material for the preparation of civetone a well known perfumery ingredient. Its conversion into civetone can be effected by means of a catalytic hydrogenation, particularly in the presence of $Pd/BaSO_4$.

The invention is better illustrated by the following non-limiting example wherein the temperatures are indicated in degrees centigrade and the abbreviations have the meaning common in the art.

## Example

Civetone

a. *Cyclohepta-2,16-dien-9-yn-1-one*

A solution of 4.44 g (20 mMole) of tetradec-7-yn-1,44-dial and 6.03 g (22 mMole) of 1,3-bis(dimethylphosphono)-propan-2-one in 30 ml of a 9:1 mixture of t-butanol and water was added to a refluxing mixture of 5 g (50 mMole) of $KHCO_3$ and 200 ml of the 9:1 mixture of t-butanol and water over a period of $6\frac{1}{2}$ h. After addition of water the mixture was extracted with ether, washed with brine, dried over $Na_2SO_4$ and evaporated. Distillation of the residue gave 2.75 g (yield 56%) of cyclohepta-2,16-dien-9-yn-1-one b.p. 128°/0.05 Torr (0.067 mbar), which solidified and was crystallized from hexane; m.p. 55—6°.

IR: 1660, 1640, 1615, 980 $cm^{-1}$;
NMR: 1.3—1.9 (12H, m), 2.0—2.6 (8H, m); 6.25 (2H, d, J=17); 6.80 (2H, d of t, J=17 and 6.5) $\delta$ ppm;
MS: $M^+ = 244$ (1); m/e: 41 (100).
UV: 240 nm ($\varepsilon = 15{,}100$).

b. *Conversion of the obtained ynone into civetone*

2.60 g (10.6 mMole) of the ynone obtained under sub letter a. above in 75 ml of pyridine was hydrogenated at 20°/760 Torr (1012 mbar) in the presence of 0.2 g of 5% $Pd/BaSO_4$ until absorption ceased (3.5 h). The mixture was filtered, evaporated and distilled to yield 2.64 g (yield 99%) of civetone b.p. 103°/0.05 Torr (0.0676 mbar);

IR: 1700 $cm^{-1}$;

Identity with authentic civetone was established by comparison of spectra and retention times on gas chromatography.

Tetradec-7-yn-1,14-dial, used as starting material in the above described process, can be prepared as follows:

i. 56 g (0.4 Mole) of cyclohexanone and enol acetate in 500 ml of methanol was ozonised in a dry ice-acetone bath until the solution turned blue. 60 ml of dimethyl sulphide was added, and the cooling bath was removed. The temperature rose slowly to 48°, and after it had returned to room temperature, 40 ml of trimethyl orthoformate and 1 ml of acetyl chloride were added. The mixture was then left for 48 h at room temperature. Three identical batches were combined and the solvent was removed in vacuo. The residue was dissolved in ether, washed with brine containing a small amount of $NaHCO_3$, dried ($Na_2SO_4$), and distilled to give 193 g of product, b.p. 56—69°/0.1 Torr (0.13 mbar). The NMR spectrum indicated the presence of ca. 20% of methyl 6-oxohexanoate. The product was mixed with 100 ml of trimethyl orthoformate, 20 ml of methanol, and 0.5 g of p-topluenesulfonic-acid. The mixture was left for 48 h at room temperature, then worked up as above yielding 201 g (88%) of methyl 6,6-dimethoxyhexanoate, b.p. 61°/0.1 Torr (0.13 mbar).

ii. A solution of 201 g (1.06 Mole) of the product obtained under sub i. in 500 ml of dry ether was added at reflux temperature within 1 h to a stirred suspension of 30 g (0.75 Mole) of $LiAlH_4$ in 750 ml of ether. Stirring was continued for 1 h at room temperature, then 100 ml of 5 N NaOH was added slowly with external cooling. Stirring became difficult, but improved toward the end. After completion of the addition stirring was continued for 1 h, then the suspension was suction filtered, washed with

ether, and evaporated. Distillation afforded 169 g (98%) of 6,6-dimethoxy-1-hexanol, b.p. 78°/0.1 Torr (0.13 mbar).

iii. 81 g (0.5 Mole) of 6,6-dimethoxy-1-hexanol was added to an ice-cooled mixture of 99.3 g (0.52 Mole) of p-toluenesulfonyl chloride and 72 ml (0.9 Mole) of pyridine at such a rate that the temperature remained below 15°. Stirring was continued for 2 h at 5—10°, then cold water was added and the mixture was extracted with ether. The organic layer was washed with 5% aq. AcOH, cold 2% NaOH, dried ($Na_2So_4$), and evaporated at 20°. The residue (190 g) was dissolved in 500 ml of acetone, then a solution of 97.5 g (0.65 Mole) of NaI in 500 ml of acetone was added, and the mixture was stirred for 24 h at room temperature. Most of the solvent was removed in vacuo, then either was added. The mixture was washed with water, dried ($Na_2SO_4$), evaporated and distilled to give 124.9 g (92%) of 1,1-dimethoxy-6-iodohexane, b.p. 76—78°/0.05 Torr (0.067 mbar).

iv. Acetyl was bubbled through ca. 300 ml of liquid ammonia while 8.1 g (0.35 atom) of sodium was added in small pieces. After the disappearance of the blue color, dimethyl sulfoxide (150 ml) was added cautiously. 1,1-Dimethoxy-6-iodohexane (81.6 g; 0.3 Mole) was then added over a period of 10 min. while acetylene was bubbled through the stirred mixture. The ammonia was allowed to evaporate and was replaced with ether. When the mixture had reached 0°, dilute $NH_4Cl$ solution was added. The organic layer was separated, washed with water, dried ($Na_2SO_4$), and evaporated. Distillation of the residue gave 48.9 g (96%) of 1,1-dimethoxy-oct-7-yne, b.p. 91°/8 Torr (10.6 mbar).

v. To a stirred suspension of lithium amide, prepared from 1.45 g (0.21 atom) of lithium in ca. 200 ml of liquid ammonia was added 30.6 g (0.18 Mole) of 1,1-dimethoxy-oct-7-yne over a period of 10 min. Dimethylsulfoxide (100 ml) was then added, followed by 62.5 g (0.23 Mole) of 1,1-dimethoxy-6-iodohexane within 10 min. Work-up as above gave an oil, from which low-boiling impurities were removed by heating at 100°/0.05 Toore (0.067 mbar). Crude tetradec-7-yn-1,14-dial di-methylacetal (40.8.g, 72% yield) was used without further purification. A sample was submitted to a rapid distillation: b.p. 140°/0.05 Torr (0.067 mbar).

IR: 1125, 1070, 1050 $cm^{-1}$

NMR: 1.1—1.8 (16H, m); 1.9—2.3 (4H, m); 3,25 (12H, s); 4.35 (2H, t, J=6) $\delta$ ppm.

vi. A mixture of 31.4 g (0.10 Mole) of the obtained diacetal, 300 ml of tetrahydrofuran, 100 ml of water, 0.5 ml of 70% perchloric acid was stirred at room temperature under argon. After 8 h a further 75 ml of water was added, and stirring was continued overnight. The mixture was poured into water, extracted with ether, washed with brine containing $Na_2CO_3$, dried ($Na_2SO_4$), and evaporated. Distillation afforded 21.1 g (95%) of tetradec-7-yn-1,14-dial, b.p. 125—127°/0.05 Torr (0.067 mbar).

IR: 2725, 1720 $cm^{-1}$

NMR: 1.1—1.8 (12H, m); 1.9—2.6 (8H, m); 9.65 (2H, t, J= ~2 Hz) $\delta$ ppm.

1,3-Bis(dimethylphosphono)-propan-2-one used as starting material in the hereinabove described process was prepared as follows:

*1,3-bis(dimethylphosphono)-2-methylene-propane*

400 ml of trimethyl phosphite was placed into a 2—1 flask equipped with an addition funnel, a gas inlet tube, and a Vigreux column with distilling head. The phosphite was heated at reflux, then a solution of 131 g (0.425 Mole) of 2-iodomethyl-3-iodopropane [see J. Amer. Chem. Soc., *89* 4688 (1967)] in 200 ml of benzene was added dropwise over a period of 2 h, followed by an additional 100 ml of trimethyl phosphite within 30 min. The reaction was run under nitrogen, and approximately 150 ml of distillate was allowed to collect. Distillation of the reaction mixture afforded 106 g (92%) of the title compound, b.p. 125—7°/0.08 Torr (0.106 mbar);

IR($CHCl_3$): 1650, 1250, 1050, 900 $cm^{-1}$;

NMR($CCl_4$): 2.75 (4H, d, J=24 Hz); 3.65 (12H, d, J=10 Hz); 5.00 (2H, t, J=5 Hz) $\delta$ ppm

MS: $M^+ = 272$ (6); m/e: 163 (100).

*1,3-bis(dimethylphosphono)-propan-2-one*

A solution of 27.2 g (0.10 Mole) of this diphosphonate obtained in 250 ml of methanol was ozonized at −10 to −20°. Trimethyl phosphite was then added dropwise at −10° (very exothermic), and the mixture was left overnight at room temperature. The solvent was removed in vacuo, and the residue was distilled to give 24.5 g (90%) of the title compound:

b.p. 156—8°/0.08 Torr (0.106 mbar);

IR($CHCl_3$): 1720, 1250, 1050 $cm^{-1}$;

NMR($CDCl_3$): 3.35 (4H, d, J=23 Hz); 3.75 (12H, d, J=11 Hz) $\varepsilon$ ppm;

MS: $M^+ = 274$ (5); m/e: 124 (100).

**Claim**

A process for the preparation of unsaturated macrocyclic ketones of formula

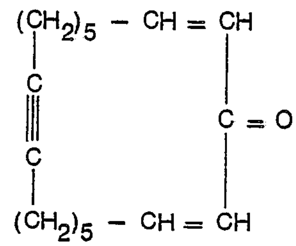

(I)

which comprises reacting in the presence of a basic reagent a dialdehyde of formula

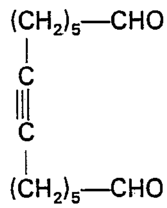

(II)

with a diphosphonate of formula

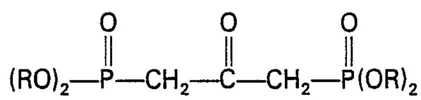

(III)

wherein each of symbols R represents a lower alkyl radical.

**Revendication**

Un procédé pour la préparation d'une cétone macrocyclique insaturée de formule

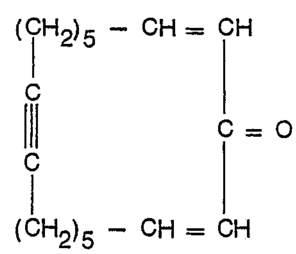

(I)

caractérisé en ce qu'on fait réagir, en présence d'un réactif basique, un dialdéhyde de formule

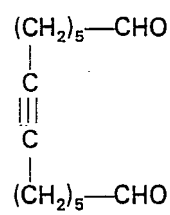

(II)

avec un diphosphonate de formule

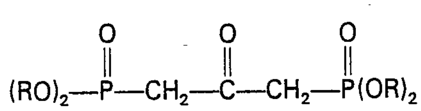

(III)

dans laquelle chacun des symbols R représente un radical alkyle inférieur.

**Patentanspruch**

Ein Verfahren zur Herstellung eines ungesättigten makrocyclischen Ketons der Formel

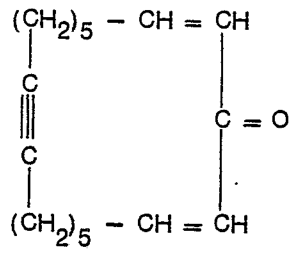

(I)

dadurch gekennzeichnet, dass ein Dialdehyd der Formel

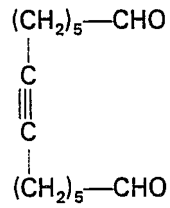

(II)

mit einem Diphosphonat der Formel

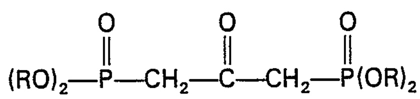

(III)

worin jedes der Symbole R ein niedriges Alkyl-Radikal bedeutet, in Anwesenheit eines basischen Reagenzes, umsetzt.